Europäisches Patentamt

(19) European Patent Office

Office européen des brevets

(11) EP 0 527 637 B1

(12) **EUROPEAN PATENT SPECIFICATION**

(45) Date of publication and mention
of the grant of the patent:
**29.04.1998 Bulletin 1998/18**

(51) Int. Cl.⁶: **A61K 31/55**, A61K 9/52,
A61K 9/54

(21) Application number: **92307333.2**

(22) Date of filing: **11.08.1992**

(54) **Spheroids containing dittiazem and a spheronizing agent**

Sphäroide enthaltend Diltiazem und Sphäronisierungsmittel

Sphéroides à base de diltiazem et d'un agent de sphéroidisation

(84) Designated Contracting States:
**AT BE CH DE DK ES FR GR IE IT LI LU MC NL PT
SE**

(30) Priority: **12.08.1991 GB 9117361
29.10.1991 GB 9122967**

(43) Date of publication of application:
**17.02.1993 Bulletin 1993/07**

(73) Proprietor: **Euroceltique S.A.
Luxembourg (LU)**

(72) Inventors:
 • **Buxton, Ian Richard
 Histon, Cambridge (GB)**
 • **Critchley, Helen
 Histon, Cambridge (GB)**
 • **Leslie, Stewart Thomas
 Cambridge (GB)**
 • **Malkowska, Sandra Therese Antoinette
 Ely, Cambridge (GB)**
 • **Prater, Derek Allan
 Milton, Cambridge (GB)**
 • **Miller, Ronald Brown
 Basle 4059 (CH)**

(74) Representative:
 **Ruffles, Graham Keith et al
 MARKS & CLERK,
 57-60 Lincoln's Inn Fields
 London WC2A 3LS (GB)**

(56) References cited:
 **EP-A- 0 315 414          EP-A- 0 322 277
 EP-A- 0 340 105          US-A- 4 808 413**

## Description

The present invention relates to a controlled release preparation and to a process for its preparation. In particular it relates to a controlled release preparation containing diltiazem.

Diltiazem is a calcium antagonist which has been shown to be useful in treating chronic heart disease such an angina and hypertension and angina.

It is an object of the present invention to provide a controlled release diltiazem preparation suitable for once daily administration for the treatment of hypertension and angina.

The present invention therefore provides a controlled release composition comprising spheroid cores consisting of 60% to 98% by weight diltiazem or a pharmaceutically acceptable salt thereof and a spheronising agent, said cores being coated with a controlled release layer.

Suitable pharmaceutically acceptable salts of diltiazem for use according to the present invention include pharmaceutically acceptable acid addition salts. The hydrochloride salt is particularly preferred.

A controlled release pharmaceutical composition according to the present invention is one that achieves slow release of a drug over an extended period of time and extends the duration of drug action over that achieved by conventional delivery.

The term "spheroid" is conventional in the pharmaceutical art and means a spherical granule having a diameter of between 0.1mm and 2.5mm, especially between 0.5mm and 2mm.

The spheroid cores for use according to the present invention preferably contain between 60% and 98%, more preferably between 60% and 85%, especially between 70% and 85% by weight of diltiazem or its pharmaceutically acceptable salts.

The spheronising agent is microcrystalline cellulose. The microcrystalline cellulose employed may be, for example, Avicel PH 101 or Avicel PH 102 (Trade Marks, FMC Corporation). Conveniently the spheronising agent is present in an amount of from 1% to 40%, preferably from 15% to 40% by weight of the spheroid cores.

Optionally the spheroid cores may also contain other pharmaceutically acceptable excipients and diluents which facilitate spheronisation and which are sugars (for example sucrose, dextrose, maltose or lactose) or sugar alcohols (for example mannitol, xylitol or sorbitol). Colourants may also be included in the spheroid core.

The spheroid cores are coated with a material which permits release of the diltiazem at a controlled rate in an aqueous medium. Suitable controlled release coating materials include those well known in the art such as water insoluble waxes and polymers such as polymethacrylates (for example Eudragit polymers, Trade Mark) or preferably water insoluble celluloses particularly ethylcellulose. The coating may also include water soluble polymers such as polyvinylpyrrolidone or preferably a water soluble cellulose such as hydroxypropylmethylcellulose and hydroxypropylcellulose. It will be appreciated that the ratio of water insoluble to water soluble material will depend on the release rate required and the solubility of the materials selected. The ratio of water soluble polymer to water insoluble polymer is preferably 1:20 to 1:2.

The controlled release coating preferably includes one or more plasticisers conventional in the art such as diethylphthalate but particularly dibutyl sebacate; surfactants such as sorbitan trioleate, sorbitan monolaurate or preferably polysorbate 80 (Tween 80, Trade Mark) and tack-modifiers such as talc or preferably colloidal anhydrous silica.

The amount of plasticiser, when present, will depend on the particular plasticiser selected. In general, the plasticiser is present in an amount of from 1% to 25% by weight of the controlled release film coat. The surfactant, when present, is suitably present in an amount of from 1% to 25% by weight of the controlled release film coat. The tack-modifier, when present, is also suitably present in an amount of from 1% to 25% by weight of the controlled release film coat.

A preferred controlled release film coating comprises 50% to 95% ethylcellulose, 5% to 15% colloidal anhydrous silica, 5% to 15% dibutyl sebacate and 5% to 15% polysorbate 80 (Tween 80, Trade Mark).

The controlled release film coating layer can be formed on the surface of the diltiazem containing spheroid cores using conventional coating methods, for example fluidised bed or pan coating. The coating materials may be applied as a solution or suspension. Suitable solvent systems include water, dichloromethane, ethanol, methanol, isopropyl alcohol and acetone or a mixture thereof. The coating solution or suspension preferably contains from 2% to 60%, preferably from 2% to 20% by weight of coating materials.

The amount of the controlled release coating material will depend on the desired release rate but is generally in the range of from 1% to 25%, preferably 2% to 8% by weight of the composition.

The controlled release composition according to the invention may be prepared by

(a) granulating a mixture consisting of diltiazem or a pharmaceutically acceptable salt thereof, water and microcrystalline cellulose, and optionally a sugar and/or a sugar alcohol and/or a colorant;
(b) extruding the granulated mixture to give an extrudate;
(c) spheronising the extrudate until spheroid cores are formed;
(d) drying the spheroid cores; and

(e) coating the spheroid cores with a controlled release material.

Compostions according to the invention may be filled into capsules or sachets or compressed into tablets using conventional pharmaceutical techniques.

The composition according to the present invention may suitably be administered once daily. Conveniently for once daily administration the dosage form contains from 120mg to 300mg of diltiazem or a pharmaceutically acceptable salt thereof, preferably diltiazem hydrochloride.

Example 1

Capsule having the following formulation were prepared

| Diltiazem spheroid cores | |
|---|---|
| Material | mg |
| Diltiazem hydrochloride U.S.P. | 120 |
| Microcrystalline cellulose E.P. (Avicel PH101) | 30.0 |
| Purified water E.P. | q.s. |
| | 150 |

| Controlled release film coat | |
|---|---|
| Material | mg |
| Diltiazem hydrochloride spheroid core | 150 |
| Ethylcellulose N10 U.S.N.F. | 7.38 |
| Colloidal anydrous silica E.P. (Aerosil 130) | 0.988 |
| Dibutyl sebacate U.S.N.F. | 0.742 |
| Polysorbate 80 E.P. (Tween 80) | 0.791 |
| Dichloromethane BS 1994 | q.s. |
| Methanol B.P. 1973 | q.s. |
| | 160 |

| Capsule formulation | |
|---|---|
| | mg |
| Diltiazem controlled release spheroids | 160 |
| Magnesium stearate EP | 0.480 |
| Gelatin capsule shells size 3 | |

The diltiazem and microcrystalline cellulose were blended using a high shear mixer. The mixture was wet granulated, and extruded to give an extrudate which was spheronised and dried in a fluid bed drier. The spheroids were sieved to give a particle size of 0.85 to 1.7mm.

The controlled release film coating ingredient were dispersed in the dichloromethane/methanol solvent system and applied to the diltiazem spheroid cores in a fluid bed coater. The resulting film coated spheroids were sieved. The coated spheroids were filed into gelatin capsule shells.

The dissolution of the resulting product was measured by EP basket apparatus at 100rpm in pH 4.5 EP phosphate buffer. The results obtained are recorded below.

| Diltiazem Dissolution | |
| --- | --- |
| Time (hours) | Diltiazem controlled release spheroids |
| 1 | 9 |
| 2 | 23 |
| 3 | 37 |
| 4 | 48 |
| 5 | 57 |
| 6 | 63 |
| 8 | 72 |
| 10 | 77 |
| 12 | 81 |
| 15 | 86 |
| 20 | 90 |

**Claims**

**Claims for the following Contracting States : AT, BE, CH, DE, DK, FR, GB, IE, IT, LI, LU, MC, NL, PT, SE**

1. A controlled release composition comprising spheroid cores consisting of 60% to 98% by weight diltiazem or a pharmaceutically acceptable salt thereof, microcrystalline cellulose and, optionally, a sugar and/or a sugar alcohol and/or a colorant, said cores being coated with a controlled release material.

2. A composition according to claim 1 wherein the spheroid cores comprise between 70% and 85% by weight of diltiazem or a pharmaceutically acceptable salt thereof.

3. A composition according to any one of claims 1 or claim 2 wherein the microcrystalline cellulose is present in an amount of from 15% to 40% by weight of the spheroid cores.

4. A composition according to any one of claims 1 to 3 wherein the controlled release coating material comprises a water insoluble polymer.

5. A composition according to claim 4 wherein the coating material comprises ethylcellulose.

6. A composition according to any one of claims 1 to 5 wherein the controlled release coating material further comprises one or more plasticisers, surfactants and tack-modifiers.

7. A composition according to claim 6 wherein the controlled release coating comprises 50% to 95% ethylcellulose, 5% to 15% colloidal anhydrous silica, 5% to 15% dibutyl sebacate and 5% to 15% polysorbate 80.

8. A composition according to any of claims 1 to 7 wherein the controlled release coating material is present in an amount of from 2% to 8% by weight of the composition.

9. A capsule comprising controlled release coated spheroid cores according to any one of claims 1 to 8.

10. A process for preparing a composition according to any one of claims 1 to 9 which comprises:

    (a) granulating a mixture consisting of diltiazem or a pharmaceutically acceptable salt thereof, water and microcrystalline cellulose, and optionally a sugar and/or a sugar alcohol and/or a colorant;

(b) extruding the granulating mixture to give an extrudate;

(c) spheronising the extrudate until spheroid cores are formed;

(d) drying the spheroid cores; and

(e) coating the spheroid cores with a controlled release material.

**Claims for the following Contracting States : ES, GR**

1. A process for preparing a controlled release composition comprising

   (a) granulating a mixture consisting of diltiazem or a pharmaceutically acceptable salt thereof, water, microcrystalline cellulose, and, optionally, a sugar and/or a sugar alcohol and/or a colorant;

   (b) extruding the granulating mixture to give an extrudate;

   (c) spheronising the extrudate until the spheroid cores are formed;

   (d) drying the spheroid cores to give cores containing from 60% to 98% by weight of diltiazem or pharmaceutically acceptable salt thereof; and

   (e) coating the spheroid cores with a controlled release material.

2. A process according to claim 2 wherein the spheroid cores comprise between 70% and 85% by weight of diltiazem or a pharmaceutically acceptable salt thereof.

3. A process according to any claim 1 or claim 2 wherein the microcrystalline cellulose is present in an amount of from 15% to 40% by weight of the spheroid cores.

4. A process according to any one of claims 1 to 3 wherein the controlled release coating material comprises a water in soluble polymer.

5. A process according to claim 4 wherein the coating material comprises ethylcellulose.

6. A process according to any one of claims 1 to 5 wherein the controlled release material further comprises one or more plasticisers, surfactants and tack-modifiers.

7. A process according to claim 6 wherein the controlled release coating comprises 50% to 95% ethylcellulose, 5% to 15% colloidal anhydrous silica, 5% to 15% dibutyl sebacate and 5% to 15% polysorbate 80.

8. A process according to any one of claims 1 to 7 wherein the controlled release coating material is present in an amount of from 2% to 8% by weight of the composition.

**Patentansprüche**

**Patentansprüche für folgende Vertragsstaaten : AT, BE, CH, DE, DK, FR, GB, IE, IT, LI, LU, MC, NL, PT, SE**

1. Zusammensetzung mit kontrollierter Abgabe, umfassend Sphäroidkerne bestehend aus 60 Gew.% bis 98 Gew.% Diltiazem oder einem pharmazeutisch verträglichen Salz davon, mikrokristalliner Cellulose und gewünschtenfalls einem Zucker und/oder einem Zuckeralkohol und/oder einem Färbemittel, wobei die Kerne mit einem Material mit kontrollierter Abgabe überzogen sind.

2. Zusammensetzung nach Anspruch 1, wobei die Sphäroidkerne zwischen 70 Gew.% und 85 Gew.% Diltiazem oder eines pharmazeutisch verträglichen Salzes davon umfassen.

3. Zusammensetzung nach einem der Ansprüche 1 oder 2, wobei die mikrokristalline Cellulose in einer Menge von 15 bis 40 Gew.% der Sphäroidkerne vorhanden ist.

4. Zusammensetzung nach einem der Ansprüche 1 bis 3, wobei das Überzugsmaterial mit kontrollierter Abgabe ein wasserunlösliches Polymer umfaßt.

5. Zusammensetzung nach Anspruch 4, wobei das Überzugsmaterial Ethylcellulose umfaßt.

6. Zusammensetzung nach einem der Ansprüche 1 bis 5, wobei das Überzugsmaterial mit kontrollierter Abgabe weiterhin ein oder mehrere Weichmacher, oberflächenaktive Mittel und Klebrigkeitsmodifikationsmittel umfaßt.

7. Zusammensetzung nach Anspruch 6, wobei der Überzug mit kontrollierter Abgabe 50% bis 95% Ethylcellulose, 5% bis 15% kolloidales wasserfreies Silika, 5% bis 15% Dibutylsebacat und 5% bis 15% Polysorbat 80 umfaßt.

8. Zusammensetzung nach einem der Ansprüche 1 bis 7, wobei das Überzugsmaterial mit kontrollierter Abgabe in einer Menge von 2 Gew.% bis 8 Gew.% der Zusammensetzung vorhanden ist.

9. Kapsel, umfassend überzogene Sphäroidkerne mit kontrollierter Abgabe nach einem der Ansprüche 1 bis 8.

10. Verfahren zum Herstellen einer Zusammensetzung nach einem der Ansprüche 1 bis 9, welches umfaßt:

(a) Granulieren einer Mischung bestehend aus Diltiazem oder einem pharmazeutisch verträglichen Salz davon, Wasser und mikrokristalliner Cellulose und gewünschtenfalls einem Zucker und/oder einem Zuckeralkohol und/oder einem Färbemittel;
(b) Extrudieren der Granuliermischung unter Erhalten eines Extrudats;
(c) Sphäroidbilden des Extrudats, bis Sphäroidkerne gebildet sind;
(d) Trocknen der Sphäroidkerne; und
(e) Überziehen der Sphäroidkerne mit einem Material mit kontrollierter Abgabe.

**Patentansprüche für folgende Vertragsstaaten : ES, GR**

1. Verfahren zum Herstellen einer Zusammensetzung mit Kontrollierter Abgabe, welches umfaßt:

(a) Granulieren einer Mischung bestehend aus Diltiazem oder einem pharmazeutisch verträglichen Salz davon, Wasser und mikrokristalliner Cellulose und gewünschtenfalls einem Zucker und/oder einem Zuckeralkohol und/oder einem Färbemittel;
(b) Extrudieren der Granuliermischung unter Erhalten eines Extrudats;
(c) Sphäroidbilden des Extrudats, bis Sphäroidkerne gebildet sind;
(d) Trocknen der Sphäroidkerne unter Erhalten von Kernen umfassend 60 Gew.% bis 98 Gew.% Diltiazem oder einem pharmazeutisch verträglichen Salz davon; und
(e) Überziehen der Sphäroidkerne mit einem Material mit kontrollierter Abgabe.

2. Verfahren nach Anspruch 1, wobei die Sphäroidkerne zwischen 70 Gew.% und 85 Gew.% Diltiazem oder eines pharmazeutisch verträglichen Salzes davon umfassen.

3. Verfahren nach einem der Ansprüche 1 oder 2, wobei die mikrokristalline Cellulose in einer Menge von 15 bis 40 Gew.% der Sphäroidkerne vorhanden ist.

4. Verfahren nach einem der Ansprüche 1 bis 3, wobei das Überzugsmaterial mit kontrollierter Abgabe ein wasserunlösliches Polymer umfaßt.

5. Verfahren nach Anspruch 4, wobei das Überzugsmaterial Ethylcellulose umfaßt.

6. Verfahren nach einem der Ansprüche 1 bis 5, wobei das Material mit kontrollierter Abgabe weiterhin ein oder mehrere Weichmacher, oberflächenaktive Mittel und Klebrigkeitsmodifikationsmittel umfaßt.

7. Verfahren nach Anspruch 6, wobei der Überzug mit kontrollierter Abgabe 50% bis 95% Ethylcellulose, 5% bis 15% kolloidales wasserfreies Silika, 5% bis 15% Dibutylsebacat und 5% bis 15% Polysorbat 80 umfaßt.

8. Verfahren nach einem der Ansprüche 1 bis 7, wobei das Überzugsmaterial mit kontrollierter Abgabe in einer Menge von 2 Gew.% bis 8 Gew.% der Zusammensetzung vorhanden ist.

**Revendications**

**Revendications pour les Etats contractants suivants : AT, BE, CH, DE, DK, FR, GB, IE, IT, LI, LU, MC, NL, PT, SE**

1. Composition à libération régulée, comprenant des coeurs sphéroïdaux constitués par 60% à 98% en poids de diltiazem ou d'un sel pharmaceutiquement acceptable de celui-ci, de la cellulose microcristalline et, facultativement, un sucre et/ou un alcool de sucre et/ou un colorant, lesdits coeurs étant revêtus avec une matière à libération régulée.

2. Composition selon la revendication 1, dans laquelle les coeurs sphéroïdaux comprennent entre 70% et 85% en poids de diltiazem ou d'un sel pharmaceutiquement acceptable de celui-ci.

3. Composition selon la revendication 1 ou la revendication 2, dans laquelle la cellulose microcristalline est présente en une proportion de 15% à 40% en poids par rapport aux coeurs sphéroïdaux.

4. Composition selon l'une quelconque des revendications 1 à 3, dans laquelle la matière de revêtement à libération régulée comprend un polymère insoluble dans l'eau.

5. Composition selon la revendication 4, dans laquelle la matière de revêtement comprend de l'éthylcellulose.

6. Composition selon l'une quelconque des revendications 1 à 5, dans laquelle la matière de revêtement à libération régulée comprend en outre un ou plusieurs plastifiants, agents tensioactifs et modificateurs d'adhésivité.

7. Composition selon la revendication 6, dans laquelle le revêtement à libération régulée comprend 50% à 95% d'éthylcellulose, 5% à 15% de silice colloïdale anhydre, 5% à 15% de sébaçate de dibutyle et 5% à 15% de polysorbate 80.

8. Composition selon l'une quelconque des revendications 1 à 7, dans laquelle la matière à libération régulée est présente en une proportion de 2% à 8% en poids par rapport à la composition.

9. Capsule comprenant des coeurs sphéroïdaux revêtus avec une matière à libération régulée selon l'une quelconque des revendications 1 à 8.

10. Procédé pour préparer une composition selon l'une quelconque des revendications 1 à 9, qui comprend:

    (a) la granulation d'un mélange constitué par du diltiazem ou un sel pharmaceutiquement acceptable de celui-ci, de l'eau et de la cellulose microcristalline et, facultativement, un sucre et/ou un alcool de sucre et/ou un colorant;
    (b) l'extrusion du mélange granulé pour donner un extrudat;
    (c) la sphéronisation de l'extrudat jusqu'à ce que des coeurs sphéroïdaux soient formés;
    (d) le séchage des coeurs sphéroïdaux; et
    (e) le revêtement des coeurs sphéroïdaux avec une matière à libération régulée.

**Revendications pour les Etats contractants suivants : ES, GR**

1. Procédé pour préparer une composition à libération régulée comprenant:

    (a) la granulation d'un mélange constitué par du diltiazem ou un sel pharmaceutiquement acceptable de celui-ci, de l'eau et de la cellulose microcristalline et, facultativement, un sucre et/ou un alcool de sucre et/ou un colorant;
    (b) l'extrusion du mélange granulé pour donner un extrudat;
    (c) la sphéronisation de l'extrudat jusqu'à ce que des coeurs sphéroïdaux soient formés;
    (d) le séchage des coeurs sphéroïdaux pour donner des coeurs contenant de 60% à 98% en poids de diltiazem ou d'un sel pharmaceutiquement acceptable de celui-ci; et
    (e) le revêtement des coeurs sphéroïdaux avec une matière à libération régulée.

2. Procédé selon la revendication 1, dans laquelle les coeurs sphéroïdaux comprennent entre 70% et 85% en poids de diltiazem ou d'un sel pharmaceutiquement acceptable de celui-ci.

3. Procédé selon la revendication 1 ou la revendication 2, dans laquelle la cellulose microcristalline est présente en une proportion de 15% à 40% en poids par rapport aux coeurs sphéroïdaux.

4. Procédé selon l'une quelconque des revendications 1 à 3, dans laquelle la matière de revêtement à libération régulée comprend un polymère insoluble dans l'eau.

5. Procédé selon la revendication 4, dans laquelle la matière de revêtement comprend de l'éthylcellulose.

6. Procédé selon l'une quelconque des revendications 1 à 5, dans laquelle la matière à libération régulée comprend en outre un ou plusieurs plastifiants, agents tensioactifs et modificateurs d'adhésivité.

7. Procédé selon la revendication 6, dans laquelle le revêtement à libération régulée comprend 50% à 95% d'éthylcellulose, 5% à 15% de silice colloïdale anhydre, 5% à 15% de sébaçate de dibutyle et 5% à 15% de polysorbate 80.

8. Procédé selon l'une quelconque des revendications 1 à 7, dans laquelle la matière de revêtement à libération régulée est présente en une proportion de 2% à 8% en poids par rapport à la composition.